(19) Europäisches Patentamt
European Patent Office
Office européen des brevets

(11) **EP 2 265 179 B1**

(12) **EUROPÄISCHE PATENTSCHRIFT**

(45) Veröffentlichungstag und Bekanntmachung des Hinweises auf die Patenterteilung:
**03.08.2016 Patentblatt 2016/31**

(51) Int Cl.:
***A61B 5/087*** *(2006.01)*   ***A61M 16/00*** *(2006.01)*

(21) Anmeldenummer: **09734116.8**

(22) Anmeldetag: **23.04.2009**

(86) Internationale Anmeldenummer:
**PCT/CH2009/000132**

(87) Internationale Veröffentlichungsnummer:
**WO 2009/129641 (29.10.2009 Gazette 2009/44)**

(54) **VORRICHTUNG UND VERFAHREN ZUR BEURTEILUNG DER BELASTUNG DES KREISLAUFS EINER PERSON WÄHREND EINER ATEMUNTERSTÜTZUNG**

APPARATUS AND METHOD FOR ASSESSING THE STRESS ON THE CIRCULATION OF A PERSON DURING ASSISTED BREATHING

DISPOSITIF ET MÉTHODE D'ÉVALUATION DU STRESS CIRCULATOIRE D'UNE PERSONNE PENDANT UNE ASSISTANCE RESPIRATOIRE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **24.04.2008 CH 650082008**

(43) Veröffentlichungstag der Anmeldung:
**29.12.2010 Patentblatt 2010/52**

(73) Patentinhaber: **Hamilton Medical AG
7402 Bonaduz (CH)**

(72) Erfinder:
• **WYSOCKI, Marc**
  **F-78460 Chevreves (FR)**
• **BRUNNER, Josef**
  **CH-7000 Chur (CH)**
• **LOPEZ GASCO, Ricardo**
  **CH-8820 Wädenswil (CH)**
• **NOVOTNI, Dominik**
  **CH-7000 Chur (CH)**
• **LAUBSCHER, Thomas**
  **CH-7403 Rhäzüns (CH)**
• **DURISCH, Gion**
  **CH-7013 Domat/Ems (CH)**

(74) Vertreter: **RLTG
Ruttensperger Lachnit Trossin Gomoll
Patent- und Rechtsanwälte
Arnulfstraße 58
80335 München (DE)**

(56) Entgegenhaltungen:
EP-A- 1 434 141     EP-A- 1 813 187
EP-A- 1 870 035     WO-A-03/077854
WO-A-2005/065540    US-A1- 2008 064 965

**Beschreibung**

Die Erfindung betrifft eine Vorrichtung gemäss Oberbegriff von Anspruch 1

Aus der EP-A 1813187 ist eine Vorrichtung zur Beurteilung des hämodynamischen Zustands eines mechanisch ventilierten Patienten bekannt. Diese ist eingerichtet, ein respiratorisches Veränderungsdiagramm einer hämodynamischen Variablen bereitzustellen und erlaubt, die Werte eines hämodynamischen Parameters für jeden mechanischen Atemzyklus abzuleiten, wie auch eine Abschätzung der Eignung dieser abgeleiteten Werte für die hämodynamische Analyse auf Basis des respiratorischen Veränderungsdiagrammes zu beurteilen. Ziel dieser Analyse ist, die Reaktion des Patienten auf eine Flüssigkeitstherapie abschätzen zu können.

Ungeeignete Werte treten beispielsweise auf, wenn der Patient an Herz-Arrhythmie leidet oder wenn der Patient unregelmässige Ahnungsmuster, d.h. eine unregelmässige respiratorische Frequenz oder ein unregelmässiges Tidal-Volumen hat.

Die Abschätzung der Eignung eines Werts wird aufgrund des Feststellens einer Arrhythmie durchgeführt. Eine solche wird bevorzugt durch die Aufzeichnung der Zeitintervalle zwischen den Puls-zu-Puls-Spitzen der hämodynamischen Variablen, oder aber mittels EKG, festgestellt. Ein Zeitintervall, bei dem der Wert des hämodynamischen Parameters geeignet ist, besitzt maximal eine vorherbestimmte Abweichung von einem mittleren Zeitintervall. Bei Zeitintervallen mit grösserer Abweichung wird der Wert des hämodynamischen Parameters von der Analyse ausgeschlossen.

Die Abschätzung der Eignung eines Werts wird auch aufgrund des Feststellens eines unregelmässigen Atmungsmusters ausgeführt. Ein solches wird aufgrund des Musters der Werte des hämodynamischen Parameters festgestellt. Die Werte des hämodynamischen Parameters, die dem mechanischen Atmungszyklusmuster zugeordnet sind, werden von der Analyse ausgeschlossen, wenn die Abweichungen im Atmungszyklus einen vorbestimmten Wert übersteigen.

Der bevorzugte hämodynamische Parameter ist die Variation des normalisierten arteriellen Blutdrucks PP. Die Variation PPV des normalisierten arteriellen Blutdrucks PPn gibt nach dieser Schrift und zum Anmeldezeitpunkt bekannter Lehre Aufschluss über eine Reaktion des Herzvolumens auf eine Flüssigkeitstherapie. Diese Variation PPV wird ermittelt nach der Formel:

$$PPV = 2 \; \frac{PPn\ max - PPn\ min}{PPn\ max + PPn\ min}$$

Die beschriebene Vorrichtung kann in direkter Verbindung mit einem Ventilator stehen. Der Ventilator sendet dann Tidal-Volumen, oder/ und Atemwegdruck, oder/ und Atemwegdurchflussmesssignale zu der Vorrichtung. Die Vorrichtung kann als hämodynamische Variable die Puls-Oximetrie-plethysmographische-Wellenform und als hämodynamischer Parameter deren Variation verwenden. Ob diese Pulsoximetrie-Signale aussagekräftig sind, war vom Erfinder zum Zeitpunkt der Veröffentlichung einschlägiger Forschungsergebnisse ("Changes in Arterial Pressure during Mechanical Ventilation" Frederic Michard, Anesthesiology 2005, Vol. 103: 419-428) noch nicht belegt.

Aus der Publikation "Does the Pleth Variability Index Indicate the Respiratory-Induced Variation in the Plethysmogram and Arterial Pressure Waveforms?" Maxime Cannesson et al., in ANESTHESIA & ANALGESIA Vol. 106, No. 4, April 2008, on pages 1190 - 1194, ist dem Fachmann bekannt, dass die Variation der Amplituden eines Plethysmogramms errechnet wird aus der minimalen und der maximalen Amplitude, die innerhalb eines gegebenen Zeitraums, nämlich einem Atemzyklus, gemessen werden.

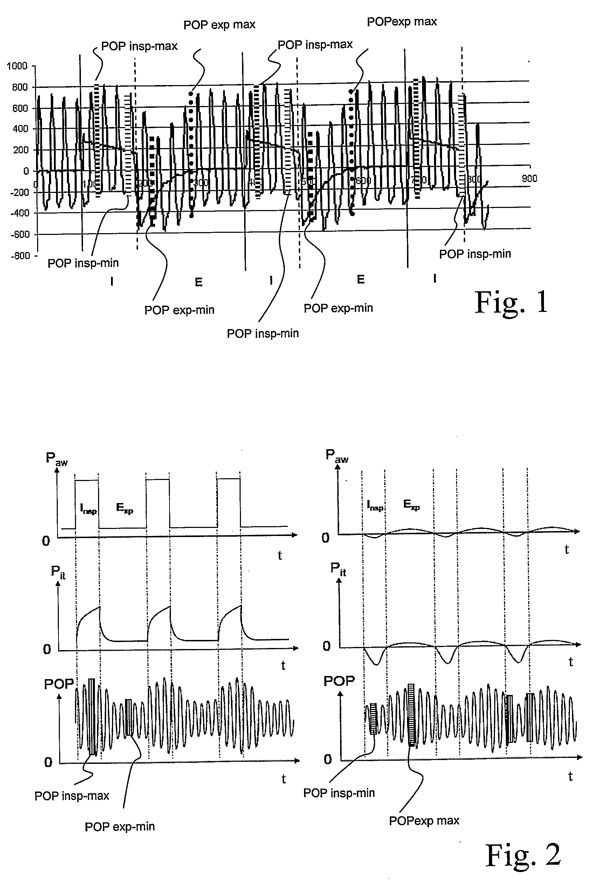

Das Dokument WO 03/077854 offenbart eine Vorrichtung mit Erfassungsmitteln, die geeignet sind zur Erfassung: der Inspirationsphase und der Exspirationsphase jedes Atemzyklus einer Person von jeweils wenigstens einer minimalen und einer maximalen Amplitude eines Zirkulationswertes innerhalb eines einzelnen Atemzyklus mit einer Recheneinrichtung zur Errechnung einer innerhalb eines besagten Atemzyklus auftretenden Variation der Amplituden des Zirkulationswertes. Wie in Fig. 1 ersichtlich, können PPmax aus der Inspirationsphase und PPmin aus der Exspirationsphase stammen, dies ist allerdings nur zufällig in den Abbildungen der Fall, die Werte werden ermittelt in einem vollständigen Atemzyklus.

Es ist daher Aufgabe der Erfindung, eine Vorrichtung zur Beurteilung der hämodynamischen Belastung einer mechanisch ventilierten Person vorzuschlagen, bei der die Korrelation der Beurteilung mit der tatsächlichen Belastung hoch ist. Es soll insbesondere eine solche Vorrichtung geschaffen werden, die die hämodynamische Belastung der Person mittels nicht-invasiver Mittel zuverlässig ermitteln kann.

Diese Aufgabe wird gelöst durch eine Vorrichtung gemäß Anspruch 1. Die erfindungsgemäße Vorrichtung kann die Belastung des Kreislaufs einer mechanisch ventilierten Person nicht invasiv und kontinuierlich beurteilen. Die Vorrichtung besitzt dazu Einrichtungen zur Ermittlung des Atemzyklus und der Variation eines für die Zirkulation repräsentativen Zirkulationswerts innerhalb des Atemzyklus. Diese kontinuierlich berechnete Variation dient als Mass für die Belastung des Kreislaufes, die stetig angezeigt werden kann, und kann als Überwachung des Kreislaufes einer mechanisch ventilierten Person verwendet werden. Des Weiteren kann diese Variation gebraucht werden, um die automatischen Einstellungsänderungen der Vorrichtung zu steuern und damit die Einstellungen stetig an den Patienten anzupassen.

Eine erfindungsgemässe Vorrichtung besitzt in bekannter Weise Erfassungsmittel, die geeignet sind zur Erfassung der Atemzyklen einer Person, insbesondere der Inspirationsphase und der Exspirationsphase jedes Atemzyklus, und die geeignet sind zur Erfassung von jeweils wenigstens einer minimalen (ZAmin) und einer maximalen Amplitude (ZAmax) eines Zirkulationswerts (Z), innerhalb eines einzelnen Atemzyklus. Sie besitzt ebenfalls in bekannter Weise eine Recheneinrichtung zur Errechnung einer innerhalb eines besagten Atemzyklus auftretenden Variation (ZAV) der Amplituden des Zirkulationswertes.

Die erfindungsgemässe Vorrichtung zeichnet sich dadurch aus, dass die Recheneinrichtung eingerichtet ist, um jede Amplitude des Zirkulationswerts der Inspirationsphase oder der Exspirationsphase zuzuordnen. Sie ist ferne dazu ausgelegt, die eine extreme, z.B. die maximale Amplitude des Zirkulationswerts aus den der Inspirationsphase zugeordneten Amplituden und die entgegengesetzt extreme, z.B. die minimale Amplitude des Zirkulationswerts aus den der Exspirationsphase zugeordneten Amplituden zu ermitteln. Ferner ist sie darauf ausgelegt, aus der Amplitudenvariation zwischen den extremen Amplituden in den beiden Phasen des Atemzyklus abzuleiten, ob die gegebene hämodynamischen Belastung durch die Atemunterstützung zu hoch ist.

Im Sinne der spezifischeren Aufgabe der Erfindung wird bevorzugt, wenn der Zirkulationswert ein Puls-Oximetrie-plethysmographischer Messwert POP ist. Wird jedoch eine invasive Messung in Kauf genommen oder ohnehin benötigt, kann der Zirkulationswert auch aus dieser Messung abgeleitet werden. Es hat sich jedoch gezeigt, dass dank der erfindungsgemässen Auswahl der Maxima und Minima der Amplituden des Zirkulationswerts die Relevanz auch der plethysmographischen Wellenform des Pulsoximetersignal erhöht werden konnte. Der Fachmann ging bisher davon aus, dass ein Maximalwert der Amplitude eines Zirkulationswerts, z.B. des arteriellen Blutdrucks, bei beatmeten Patienten immer innerhalb der Inspirationsphase und der Minimalwert der Amplitude immer innerhalb der Exspirationsphase liegt (vergl. notes related to Fig. 5. of "Using heart-lung interactions to assess fluid responsiveness during mechanical ventilation", Crit Care 2000, 4:282-289,1 September 2000). Bei Versuchen der Anmelderin an mechanisch beatmeten Lebewesen wurde die Korrelation der Puls-Oximetrie-plethysmographischen Wellenform mit dem Herz-Ausstossvolumen beobachtet. Es hat sich gezeigt, dass die oben angeführte Annahme nicht immer stimmt. Bei mechanisch beatmeten Personen können innerhalb der Exspirationsphase grössere Amplituden des Zirkulationswerts auftreten als innerhalb der Inspirationsphase (Fig.1).

Eine höhere Korrelation zwischen der Variation der Amplitude des Zirkulationswerts und dem Herz-Ausstossvolumen konnte erreicht werden, indem die maximalen Amplituden nur innerhalb der Inspirationsphase und die minimalen Amplituden nur innerhalb der Exspirationsphase eruiert wurden. Die Zuordnung jeder maximalen und minimalen Amplitude zu einer Inspirations- oder Expirationsphase entspricht der Änderung der Druckverhältnisse innerhalb des Brustkorbes zwischen den zwei Phasen des Atemzugs. Durch diese Zuordnung wird eine Filterung eingebaut, die hilft, künstlich, z.B. durch die Bewegung des Patienten, entstehende Situationen zu finden, wo die minimale und maximale Amplitude in der gleichen Atemzugsphase entstehen. Da solche Situationen in Praxis sehr oft vorkommen können, wird die auf dieser Zuordnung basierende Berechnung deutlich robuster im Vergleich zu einer Berechnung die es erlaubt, die minimale und maximale Amplitude mit der gleichen Atemzugsphase zu assoziieren.

Vorteilhaft ermittelt die Recheneinheit nicht nur eine der beiden möglichen extremen Amplituden jeder Atemphase, sondern gleich beide. Die Recheneinheit ist daher in einer vorteilhaften Ausführungsform so ausgelegt, dass sie sowohl in der Exspirationsphase als auch in der Inspirationsphase je eine minimale und eine maximale Amplitude ermittelt. Weiter ist sie so eingerichtet, dass sie aus diesen vier Werten pro Atemzyklus beide Amplitudenvariationen errechnet, und zwar zwischen der minimalen Amplitude in der Inspirationsphase und der maximalen Amplitude in der Exspirationsphase, und zwischen der maximalen Amplitude in der Inspirationsphase und der minimalen Amplitude in der Exspirationsphase. Die Recheneinheit ist in diesem Fall zweckmässigerweise so ausgelegt, dass sie von den beiden errechneten die betragsmässig grössere Amplitudenvariation des Atemzyklus sowie deren Vorzeichen als aussagekräftig für die hämodynamische Belastung nimmt. Bei einem aktiv atmenden Patienten kann es nämlich sein, dass die Variation genau umgekehrt ausfällt als bei einem passiven Patienten (siehe Fig. 2). Dann ist die grössere der beiden Variationen nämlich die Variation zwischen der maximalen Amplitude in der Exspirationsphase und der minimalen Amplitude in der Inspirationsphase.

Eine solche Vorrichtung wäre dann ausgebildet, um während den Atemzügen die maximale und minimale Amplitude des Zirkulationswerts aus den der Exspirationsphase zugeordneten Amplituden und die maximale und minimale Amplitude des Zirkulationswerts aus den der Inspirationsphase zugeordneten Amplituden zu ermitteln, und aus der Varianz dieser Amplituden einen Wert ($\alpha$) zu errechnen, der repräsentativ für die hämodynamische Belastung der Person ist. Eine solche, die plethysmographische Wellenform des Pulsoxymeter-Signals verarbeitende Vorrichtung ist einzigartig, da sie den hämodynamischen Zustand von aktiv atmenden und passiv beatmeten Patienten zu beurteilen erlaubt.

Die Recheneinheit ist so ausgelegt, dass während der Beatmung der Wert ($\alpha$) kontinuierlich und nicht invasiv berechnet und angezeigt wird. Es wird kein externer Stimulus, Manouver oder Änderung der normalen Beatmung benötigt, um die Kreislaufbelastung des Patienten mittels α zu bestimmen

Im Zusammenhang mit mechanisch beatmeten Personen ist vor allem von Interesse, den Einfluss der Beatmung auf den Kreislauf unter Kontrolle zu halten. Es ist daher eine Vorrichtung bevorzugt, bei der die Recheneinrichtung ausgebildet ist, um die Variation der Amplituden der Zirkulationswerte eines Atemzyklus als Grundlage für die Ermittlung eines angepassten endexspiratorischen Druckes PEEP zu verwenden und diesen zu verwendenden PEEP einer maschinellen Beatmung vorzugeben. Ist die Vorrichtung im Stande, aufgrund der Veränderung des plethysmographischen Pulsoximetersignals auf die Beeinflussung des Herz-Ausstossvolumens durch den gegebenen PEEP zu schliessen, kann die Wirkung der Beatmung optimiert werden, indem der PEEP optimiert wird. Dazu sind mit Vorteil Grenzwerte der maximal zulässigen Variation der Amplitude des Zirkulationswerts festgelegt und von der Recheneinheit zu verwenden. Diese Grenzwerte können altersabhängig, konstitutionsabhängig, und/ oder vorzugsweise abhängig vom Gesundheitszustand der Lunge (z.B. Compliance) und der Blutgefässe (z.B. Arteriosklerose) festgelegt sein.

Die Recheneinheit ist zweckmässigerweise so ausgebildet, dass sie aufgrund der Variation der Amplituden des Zirkulationswerts eines Atemzyklus und durch Vergleich mit gespeicherten Grenzwerten entscheidet, ob der PEEP angehoben werden kann oder abgesenkt werden muss.

Der PEEP-Entscheidung ist vorteilhaft eine gemittelte Variationen der Amplituden ZA des Zirkulationswerts Z von aufeinanderfolgenden, bei der PEEP-Entscheidung berücksichtigten Atemzyklen zugrunde gelegt. Ein bevorzugte Mittlung ist die Bildung des Medians ZAVmed aus mehreren ermittelten Variationen ZAV. Die verwendete Anzahl der berücksichtigten Atemzyklen ist in der Recheneinheit gespeichert, kann aber geändert werden.

Die für die Beurteilung der hämodynamischen Belastung oder die Ermittlung eines angemessenen PEEP zu verwendende Variation ZAV der Amplitude ZA des Zirkulationswerts Z eines Atemzyklus ist zweckmässigerweise normalisiert, verallgemeinert insbesondere nach der Formel:

oder

$$ZAV1 = 2 \frac{ZA\ insp\text{-}max\ -\ ZA\ exp\text{-}min}{ZA\ insp\text{-}max\ +\ ZA\ exp\text{-}min} \qquad \text{(Gleichung 1a)}$$

$$ZAV2 = 2 \frac{ZA\ insp\text{-}min\ -\ ZA\ exp\text{-}max}{ZA\ insp\text{-}min\ +\ ZA\ exp\text{-}max} \qquad \text{(Gleichung 1b)}$$

oder spezifisch auf das Pulsoxymetersignal abgestellt nach der Formel: oder

$$POPV1 = 2 \frac{POP\ insp\text{-}max\ -\ POP\ exp\text{-}min}{POP\ insp\text{-}max\ +\ POP\ exp\text{-}min} \qquad \text{(Gleichung 2a)}$$

$$POPV2 = 2 \frac{POP\ insp\text{-}min\ -\ POP\ exp\text{-}max}{POP\ insp\text{-}min\ +\ POP\ exp\text{-}max} \qquad \text{(Gleichung 2b)}$$

Es kann auch ein Prozentwert durch Multiplikation des oben dargestellten Werts mit 100 verwendet werden.

Aus den beiden Werten ZAV1 und ZAV2 bzw. POPV1 und POPV2 wird der betragsmässig grössere genommen. Der betragsmässig höhere Wert wird als relevant für die Aussage genommen, ob die hämodynamische Belastung durch die Beatmung verträglich oder zu hoch ist. Dazu wird der betragsmässig höhere Wert mit einer von PEEP abhängigen Konstanten k verglichen. Ist der Wert unter Berücksichtigung des Vorzeichens höher als k, so ist die hämodynamische Belastung zu hoch. Ist dieser Wert unter Berücksichtigung des Vorzeichens niedriger als k oder gleich k, so ist die Belastung im Rahmen des Zumutbaren. Es sind bei einer bevorzugten Ausführungsform der Erfindung auch Anzeigemittel vorhanden. Diese zeigen die Amplitudenvariation an, oder sie zeigen die Ableitung aus der Amplitudenvariation an, die angibt, ob die hämodynamische Belastung durch die Beatmung für die Person zu hoch ist oder nicht. Die berechnete Amplitudenvariation oder Ableitung aus der Amplitudenvariation kann mit einem gespeicherten Grenzwert verglichen und beim Überschreiten dieses Grenzwerts als optisches oder akustisches Signal angezeigt werden. Dadurch wird beispielsweise das medizinische Personal stetig über den hämodynamischen Zustand des Patienten informiert. Im Falle einer ungünstigen Veränderung des Kreislaufes ist somit das medizinische Personal rechtzeitig alarmiert. Gegebenenfalls können die Anzeigemittel einen Trend einer dieser Grössen angeben. Die Anzeigemittel können auch mehrere der angeführten Werte anzeigen.

Die erfindungsgemässe Vorrichtung kann ein Beatmungsgerät sein. In diesem Falle sind die Erfassungsmittel zur Erfassung des Atemzyklus und der Atemphasen in dem Beatmungsgeräts integriert. Vorteilhaft umfasst das Beatmungsgerät einen Pulsoxymeter als Sensor zur Ermittlung des Zirkulationswerts.

Die Vorrichtung kann aber auch eine Überwachungseinrichtung ohne Beatmungsfunktion sein. Dann umfasst sie eine Überwachungsvorrichtung, ein Pulsoxymeter und an die Überwachungsvorrichtung angeschlossene Erfassungsmittel zur Erfassung des Atemzyklus und der Atemphasen.

Es kann vorkommen, dass in die Inspirations- oder Exspirationsphase zu wenige Pulse fallen, um eine minimale und maximale Amplitude zu ermitteln. Eine Möglichkeit, um auf die geschilderte Situation zu reagieren, um verwendbare Resultate zu erhalten besteht darin, über mehrere Inspirationsphasen und mehrere Exspirationsphasen lediglich einen Maximalwert und einen Minimalwert zu eruieren und diese Variation innerhalb mehrerer Atemzyklen für die Auswertung, zur Bestimmung der hämodynamischen Belastung des Patienten, zu verwenden. Insbesondere werden Atemphasen ohne Amplitude nicht berücksichtigt. Dazu ist es erforderlich, dass die Recheneinheit die Amplituden über mehrere Atemzyklen jeweils den Inspirationsphasen oder den Exspirationsphasen zuordnet und den einen Extremwert der Amplituden aus den den Inspirationsphasen zugeordneten Amplituden und den anderen Extremwert aus den den Exspirationsphasen dieser mehreren Atemzyklen zugeordneten Amplituden ermittelt.

Die Erfindung betrifft auch ein Verfahren. Das Verfahren umfasst folgende bekannten Schritte:

- Das Erfassen der Atemzyklen einer Person und der Inspirationsphase und der Exspirationsphase jedes Atemzyklus;
- das Erfassen von jeweils wenigstens einer minimalen und einer maximalen Amplitude eines Zirkulationswerts innerhalb eines einzelnen Atemzyklus;
- das Berechnen einer innerhalb eines besagten Atemzyklus auftretenden und für einen hämodynamischen Zustand der Person repräsentativen Variation der Amplituden des Zirkulationswertes.

Das Verfahren zeichnet sich durch den Schritt der Zuordnung jeder Amplitude des Zirkulationswerts zu der Inspirationsphase oder der Exspirationsphase, und die anschliessende Ermittlung der maximalen und minimalen Amplituden des Zirkulationswerts innerhalb einer bestimmten Atemphase aus. Bei einer mit einem Beatmungsdruck beatmeten Person geschieht die Ermittlung der maximalen Amplitude des Zirkulationswerts aus den der Inspirationsphase zugeordneten Amplituden und die Ermittlung der minimalen Amplitude des Zirkulationswerts aus den der Exspirationsphase zugeordneten Amplituden. Bei aktiv atmenden Personen hingegen geschieht die Ermittlung der maximalen Amplitude des Zirkulationswerts umgekehrt aus den der Exspirationsphase zugeordneten Amplituden und die Ermittlung der minimalen Amplitude des Zirkulationswerts aus den der Inspirationsphase zugeordneten Amplituden.

Die Erfindung wird nachfolgend anhand der Figuren im Detail erläutert. Es zeigt schematisch:

Fig. 1   ein Pulsoxymeter-Plethysmogramm mit zeitlich korreliertem Flussdiagramm einer mechanisch beatmeten Person.

Fig. 2   eine Gegenüberstellung von Druckdiagrammen und Plethysmogramm bei einem mechanisch beatmeten und bei einem aktiv atmenden, hämodynamisch instabilen Patienten.

Fig. 3   ein Flussdiagramm des Verfahrens.

Fig. 4   eine an ein erfindungsgemässes Beatmungsgerät angeschlossene Person.

Die in der Figur 1 diagrammatisch dargestellten Atemzyklen einer mittels positivem Beatmungsdruck mechanisch beatmeten Person sind durch die Flusskurve des Atemgases dargestellt. Mit einer ausgezogenen Linie ist der Beginn der Inspirationsphase und mit einer unterbrochenen Linie der Beginn der Exspirationsphase markiert. Über diese Flusskurve ist das Plethysmogramm eines Pulsoxymeters gelegt. Die plethysmographische Wellenform korreliert mit der Flusskurve. In ähnlicher Weise korreliert sie auch mit einer hier nicht dargestellten Druckkurve, sei diese auf den Druck des Beatmungsgases oder auf einen Thorax-Innendruck bezogen.

Das Plethysmogramm zeichnet eine Wellenform mit überlagerten Wellen. Die eine der Wellen zeigt die Pulsfrequenz, die andere die Atemfrequenz. Die in der Pulsfrequenz vorhandenen Maxima des Plethysmogramms steigen in der Atemfrequenz im Laufe der Exspirationsphase und zu Beginn der Inspirationsphase an und fallen im Laufe der Inspirationsphase und zu Beginn der Exspirationsphase wieder ab. Dieser Anstieg und Abfall erfolgt bei den Minima jedoch nicht parallel dazu. Die Amplitude zwischen den in Pulsfrequenz auftretenden Minima und Maxima ist nicht konstant, sondern abhängig vom Beatmungsdruck, insbesondere vom PEEP, und von der hämodynamische Stabilität der beatmeten Person. Bei hämodynamisch instabilen Personen ist die Variation der Amplituden grösser als bei hämodynamisch stabilen Personen. Bei höherem PEEP ist die Variation der Amplituden grösser als bei niedrigerem PEEP. Ähnlich wie bei den Amplituden des arteriellen Blutdruckes, wo eine Amplitudenvariation Aufschluss über die Empfänglichkeit des Patienten bezüglich einer Flüssigkeitstherapie gibt, gibt diese Amplitudenvariation im Plethysmogramm Aufschluss über die hämodynamischen Belastung des Patienten durch die mechanische Beatmung.

Es hat sich gezeigt, dass entgegen der bisherigen Annahme, die maximale Amplitude innerhalb eines mechanisch beatmeten Atemzyklus nicht immer in der Inspirationsphase liegt und die minimale Amplitude auch mal in der Inspirationsphase liegen kann. In der Figur 1 liegen zum Beispiel sowohl die absolut maximale Amplitude (POP exp-max) als auch die absolut minimale Amplitude (POP exp-min) innerhalb der Exspirationsphase. Erfindungsgemäss werden nicht die beiden absoluten Werte zur Bildung der für die hämodynamische Beurteilung der Person gebildete Variation verwendet. Sondern es werden die maximale Amplitude innerhalb der Inspiration POP insp-max und die minimale Amplitude innerhalb der Exspiration POP exp-min, die in diesem Fall identisch mit der absolut minimalen Amplitude ist, verwendet. Die Verwendung der auf die einzelnen Phasen bezogenen Werte ergibt eine grössere Korrelation zwischen der Variation der Amplituden des Plethysmogramms und den Herz-Ausstossvolumen als alleine die Verwendung der absolut maximalen und minimalen Amplituden.

Im in Figur 1 dargestellten Fall ist rechts der aktuelle, unvollständige Atemzyklus dargestellt. Die Berechnung des POPV erfolgt nach Abschluss eines Atemzyklus, so dass im dargestellten Zeitpunkt zumindest für den links dargestellten vorletzten Atemzyklus die POPV-Berechnung vorliegt.

In der Figur 2 sind sechs Diagramme gezeigt, nämlich jeweils übereinander drei schematische Diagramme zu einem mechanisch beatmeten Patienten auf der linken Seite und drei schematische Diagramme zu einer spontan und aktiv atmenden Person auf der rechten Seite. Die obersten Diagramme zeigen den Atemwegsdruck P aw über drei Atemzyklen, die mittleren Diagramme zeigen schematisch einen Thorax-Innendruck P it, zum Beispiel den Pleuraldruck, über diese drei Atemzyklen. Die untersten Diagramme zeigen das pulsoximetrische Plethysmogramm POP über dieselben Atemzyklen. Die drei Kurven in den zusammengehörigen Diagrammen sind zeitlich korreliert. Die Grenzen zwischen Inspirationsphase I nsp und Exspirationsphase E xp sind durch unterbrochene Linien markiert, die sich über alle drei Diagramme erstrecken.

In den POP darstellenden Diagrammen ist die plethysmographische Wellenform eingezeichnet. Darin sind die maximalen Amplituden POP insp-max innerhalb der Inspirationsphase und die minimalen Amplituden POP exp-min innerhalb der Exspirationsphase durch vertikal schraffierte Streifen markiert. Bei einer mechanisch beatmeten und hämodynamisch instabilen Person ist die Differenz von POP insp-max minus POP exp-min gross und positiv. Bei einer aktiv atmenden und hämodynamische instabilen Person ergeben sich jedoch Werte um Null. Wird hingegen in der Inspirationsphase die minimale Amplitude POP insp-min und in der Exspirationsphase die maximale Amplitude POP exp-max bestimmt, so ergibt sich wieder ein relativ grosser aber negativer Wert aus POP insp-min minus POP exp-max. Diese letzten beiden Amplituden sind durch horizontal schraffierte Streifen markiert. Nach Auffassung der Erfinder ist die betragsmässig grössere Variation der oben beschriebenen die aussagekräftigere, unabhängig davon, ob die Person aktiv oder passiv ist.

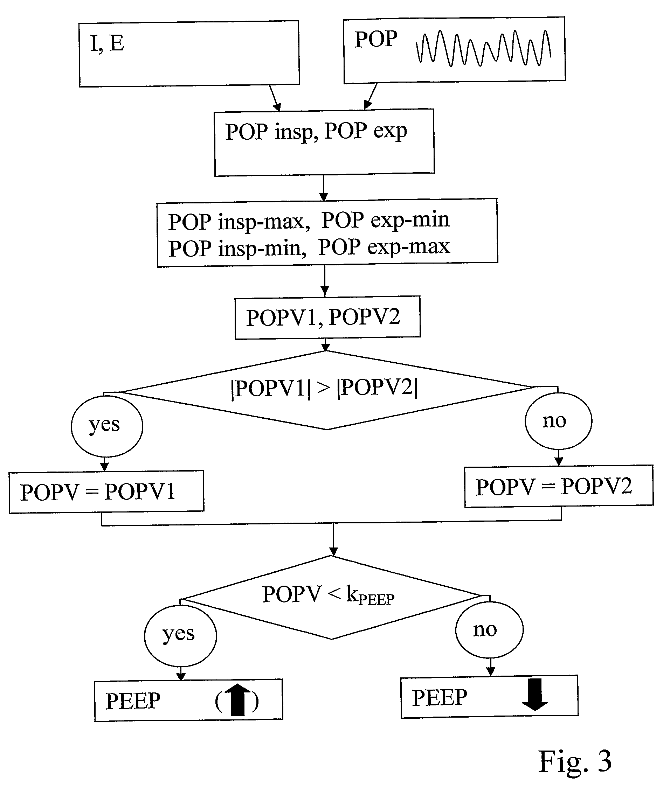

Das in Figur 3 dargestellte Flussdiagramm illustriert den Ablauf des von der Recheneinheit der Vorrichtung durchgeführten Entscheidungsverfahrens. Es wird bei jedem Atemzyklus zuerst die Inspirationsphase I und danach die Exspirationsphase E detektiert. Gleichzeitig wird das Plethysmogramm aufgezeichnet. Dann werden, in einer einfachen Ausführungsform, die maximale Amplitude POP insp-max innerhalb der Inspirationsphase I und die minimale Amplitude POP exp-min innerhalb der Exspirationsphase E bestimmt und daraus beispielsweise nach Gleichung 2a POPV, das heisst die Variation dieser Amplituden bestimmt. Dann wird diese Variation verglichen mit einer PEEP abhängigen Konstante k. Ist POPV kleiner als diese, einen Grenzwert für POPV darstellende Konstante k, so darf der verwendete PEEP erhöht werden, ist POPV grösser gross wie k, so muss der PEEP reduziert werden. Es ist auch möglich einen Bereich um k festzulegen, innerhalb welchem PEEP belassen wird.

In einer bevorzugten Ausführungsform werden sowohl die maximale als auch die minimale Amplitude in jeder Phase bestimmt und jeweils die maximale oder minimale Amplitude in der Inspirationsphasen minus die minimale oder maximale Amplitude in der Exspirationsphase gerechnet. Danach wird die betragsmässig grössere Amplitudenvariation ermittelt und das Vorzeichen der betragsmässig grösseren Amplitude berücksichtig. Danach wird wie oben beschrieben vorgegangen, wobei der positive oder negative Wert der betragsmässig grösseren Amplitudenvariation dazu verwendet wird. Der Vergleich der Amplitudenvariation mit der vom gegenwärtig eingestellten PEEP abhängigen Konstanten k ergibt, ob der PEEP bei Bedarf erhöht werden darf (Pfeil in Klammern gesetzt), oder ob er erniedrigt werden muss. Eine negative Amplitudenvariation, bei aktiv atmender Person, ist indes nicht ein Grund zur Reduktion von PEEP, da dieser Wert immer unter k liegt.

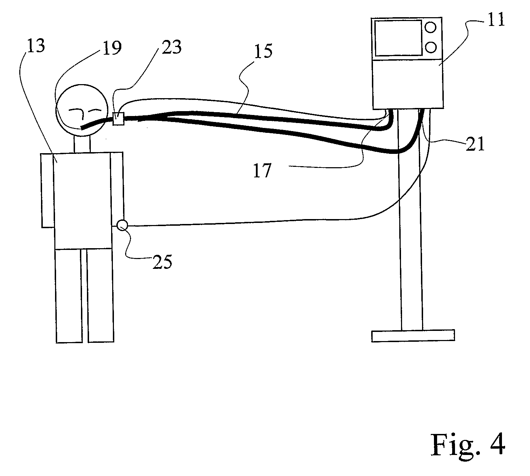

Das in Figur 4 dargestellte Beatmungsgerät 11 ist angeschlossen an eine beatmete Person 13. Ein y-förmiger Beatmungsschlauch 15 verbindet einerseits die Druckseite des Beatmungsgeräts 11, bzw. den Ausgang 17 nach dem Inspirationsventil mit dem Mundstück 19, und andererseits das Mundstück 19 mit dem Anschluss 21 zum Exspirationsventil im Beatmungsgerät 11. Im Beatmungsgerät und im Beatmungsschlauch 15 sind Ventile und Druck- und/oder Fluss-Sensoren, z.B. 23, angeordnet. Diese liefern die Information über Inspirationsphasen und Exspirationsphasen und damit über die Atemzyklen. Das Beatmungsgerät liefert die für die Beatmung notwendigen Drücke und Volumina im Rhythmus der Atemzyklen.

Ein Pulsoxymeter 25 ist an der Hand des Patienten 13 angeordnet und mit dem Beatmungsgerät 11 verbunden. Die Signale des Pulsoximeters werden durch den Rechner des Beatmungsgeräts 11 mit den Informationen über die Atemphasen zeitlich korreliert und deren Frequenz und Amplituden analysiert. Es werden verwendbare minimalen und maximalen Amplituden aus den beiden Atemphasen ermittelt und gemäss den Gleichungen 2a/2b und einem Vergleich der beiden erhaltenen Amplitudenvariationen die zur weiteren Verwendung geeignete Amplitudenvariation POPV ermittelt. Aufgrund des erhaltenen POPV und einem Grenzwert k wird entschieden, ob der vorhandene PEEP erhöht werden darf, auf einem maximalen Wert liegt oder reduziert werden muss. Gegebenenfalls werden dieser Entscheidung auch die oder einzelne von patientenspezifischen Werten, die im Beatmungsgerät erfasst sind, zugrunde gelegt, bzw. der Grenzwert k gegebenenfalls entsprechend diesen patientenspezifischen Werten bemessenen.

Wird demnach aufgrund des plethysmographischen Signals des Pulsoxymeters und den Atemphasen festgestellt, dass das Herz-Ausstossvolumen zu stark beeinträchtigt wird durch die Beatmung, so wird der PEEP solange reduziert, bis die Wellenform des Plethysmogrammes dies nicht mehr erfordert. Ist der Patient jedoch hämodynamische stabil, so beeinflusst der PEEP die plethysmographische Wellenform nur gering und der PEEP kann, sofern aus beatmungstechnischen Überlegungen heraus gewünscht, über den derzeit angewendeten PEEP erhöht werden.

Bei der PEEP-Entscheidung, bei der Triggerung der Atemzyklen, bei der Erfassung der Wirksamkeit der Ventilation und der O2-Versorgung können, abgesehen von der erfindungsgemässen Analyse der hämodynamischen Belastung durch die wechselnden Drücke im Innern des Thorax, bekannte Abläufe und bekannte Vorrichtungen zum Einsatz kommen.

## Patentansprüche

1. Vorrichtung (11) mit Erfassungsmitteln (23, 25), die geeignet sind zur Erfassung

   - der Inspirationsphase und der Exspirationsphase jedes Atemzyklus einer beatmeten Person (13),
   - von jeweils wenigstens einer minimalen und einer maximalen Amplitude eines Zirkulationswerts innerhalb eines einzelnen Atemzyklus,
   und mit einer Recheneinrichtung zur Errechnung
   - einer innerhalb eines besagten Atemzyklus auftretenden Variation der Amplituden des Zirkulationswertes,
   **dadurch gekennzeichnet,**
   **dass** die Recheneinrichtung eingerichtet ist,
   - um jede Amplitude des Zirkulationswerts entweder der Inspirationsphase (I) oder der Exspirationsphase (E) zuzuordnen, und
   - um die eine extreme Amplitude des Zirkulationswerts aus den der Inspirationsphase zugeordneten Amplituden und die andere extreme Amplitude des Zirkulationswerts aus den der Exspirationsphase zugeordneten Amplituden zu ermitteln, und
   - um aus der Amplitudenvariation zwischen den extremen Amplituden in den beiden Phasen des Atemzyklus abzuleiten, ob die gegebene hämodynamisch Belastung durch die Atemunterstützung zu hoch ist.

2. Vorrichtung nach Anspruch 1, **dadurch gekennzeichnet, dass** die Recheneinheit derart eingerichtet ist, um sowohl in der Inspirationsphase als auch in der Exspirationsphase jeweils eine maximale und jeweils eine minimale Amplitude zu ermitteln, und beide Amplitudenvariationen zwischen der maximalen Amplitude in einer Phase und der minimalen Amplitude in der anderen Phase zu ermitteln.

3. Vorrichtung nach Anspruch 2, **dadurch gekennzeichnet, dass** die Recheneinheit ausgebildet ist, um von den ermittelten Amplitudenvariationen die betragsmässig grössere unter Berücksichtigung von deren Vorzeichen als den für die hämodynamische Belastung der Person relevanten Wert zu nehmen.

4. Vorrichtung nach einem der Ansprüche 1 bis 3, **gekennzeichnet durch** Anzeigemittel für die Anzeige der Amplitudenvariation und/ oder die Ableitung aus der Amplitudenvariation, die angibt, ob die hämodynamischen Belastung **durch** die Beatmung für die Person zu hoch ist oder nicht, und/oder eines Trends wenigstens einer dieser Grössen.

5. Vorrichtung nach einem der Ansprüche 1 bis 4, **dadurch gekennzeichnet, dass** die Recheneinrichtung ausgebildet ist, um aufgrund der Variation der Amplituden der Zirkulationswerte eines Atemzyklus zu entscheiden, ob ein endexspiratorischer Druck PEEP des Beatmungsgeräts angehoben werden darf oder abgesenkt werden muss.

6. Vorrichtung nach den Ansprüchen 2 und 5, **dadurch gekennzeichnet, dass** die betragsmässig grössere Variation der Amplituden des Zirkulationswerts unter Berücksichtigung des Vorzeichens bei der PEEP-Entscheidung berücksichtigt wird.

7. Vorrichtung nach einem der Ansprüche 5 oder 6, **dadurch gekennzeichnet, dass** der PEEP-Entscheidung ein Median ZAVmed von mehreren, insbesondere elf, Variationen ZAV der Amplituden ZA des Zirkulationswerts Z von aufeinanderfolgenden, in der PEEP- Entscheidung berücksichtigten Atemzyklen zugrunde gelegt ist.

8. Vorrichtung nach einem der Ansprüche 1 bis 7, **dadurch gekennzeichnet, dass** die Variation ZAV der Amplitude ZA des Zirkulationswerts Z eines Atemzyklus normalisiert ist, insbesondere nach der Formeln:

$$ZAV1 = 2\ \frac{ZA\ insp\text{-}max\ -\ ZA\ exp\text{-}min}{ZA\ insp\text{-}max\ +\ ZA\ exp\text{-}min}$$

bzw.

$$ZAV2 = 2\ \frac{ZA\ insp\text{-}min\ -\ ZA\ exp\text{-}max}{ZA\ insp\text{-}min\ +\ ZA\ exp\text{-}max}$$

wobei ZA insp-max die maximale Amplitude eines Zirkulationswerts in der Inspirationsphase, ZA exp-min die minimale Amplitude eines Zirkulationswerts in der Exspirationsphase, ZA insp-min die minimale Amplitude eines Zirkulationswerts in der Inspirationsphase und ZA exp-max die maximale Amplitude eines Zirkulationswerts in der Exspirationsphase ist.

9. Vorrichtung nach einem der Ansprüche 1 bis 8, **dadurch gekennzeichnet, dass** der Zirkulationswert Z ein Puls-Oximetrie-plethysmographischer Messwert POP ist.

10. Vorrichtung nach einem der Ansprüche 1 bis 9, **dadurch gekennzeichnet, dass** die Erfassungsmittel zur Erfassung der Atemphasen in einem Beatmungsgerät (11) integriert sind.

11. Vorrichtung nach Anspruch 10, **dadurch gekennzeichnet, dass** die Vorrichtung das Beatmungsgerät und ein an das Beatmungsgerät angeschlossenes Pulsoxymeter (25) umfasst.

12. Vorrichtung nach Anspruch 11, **dadurch gekennzeichnet, dass** die Recheneinrichtung eingerichtet ist, um über mehrere Inspirationsphasen und Exspirationsphasen lediglich einen Maximal- und einen Minimalwert der Amplituden zu eruieren und die Variation der Amplituden innerhalb dieser Atemzüge für die Auswertung zu verwenden.

13. Vorrichtung nach einem der Ansprüche 1 bis 12, **dadurch gekennzeichnet, dass** die Recheneinrichtung eingerichtet ist,

    - um aus mehreren Atemzyklen die Amplituden den Inspirationsphasen oder den Exspirationsphasen zuzuordnen und
    - um die eine extreme Amplitude des Zirkulationswerts aus den den mehreren Inspirationsphasen zugeordneten Amplituden und die andere extreme Amplitude des Zirkulationswerts aus den den mehreren Exspirationsphasen zugeordneten Amplituden zu ermitteln.

14. Vorrichtung nach einem der Ansprüche 1 bis 13, **dadurch gekennzeichnet, dass** die Vorrichtung eine Überwachungsvorrichtung, ein an diese angeschlossenes Pulsoxymeter und an die Überwachungsvorrichtung angeschlossene Erfassungsmittel zur Erfassung der Atemphasen umfasst.

15. Verfahren, umfassend:

    - Erfassen der Inspirationsphase und der Exspirationsphase jedes Atemzyklus einer Person mittels Erfassungsmitteln (23, 25)
    - Erfassen von jeweils wenigstens einer minimalen und einer maximalen Amplitude eines Zirkulationswerts innerhalb eines einzelnen Atemzyklus,

- Errechnen einer innerhalb eines besagten Atemzyklus auftretenden Variation der Amplituden des Zirkulationswertes,
**gekennzeichnet durch**
- die Zuordnung jeder Amplitude des Zirkulationswerts zu der Inspirationsphase oder der Exspirationsphase mittels einer Rechnereinrichtung, und
- die Ermittlung der einen extremen Amplitude des Zirkulationswerts aus den der Inspirationsphase zugeordneten Amplituden und der anderen extremen Amplitude des Zirkulationswerts aus den der Exspirationsphase zugeordneten Amplituden mittels der Recheneinrichtung.

**Claims**

1. Device (11) with detection means (23, 25) which are suitable for the detection

    - of the inspiratory phase and expiratory phase of each respiratory cycle of a ventilated person (13),
    - of at least one minimum and one maximum amplitude of a circulation value within one single respiratory cycle, and with a calculation device for calculating
    - a variation of the amplitudes of the circulation value occurring during said respiratory cycle,
    **characterized by** the calculation device being adapted
    - for attributing each amplitude of the circulation value either to the inspiratory phase (I) or to the expiratory phase (E), and
    - for determining the one extreme amplitude of the circulation value out of the amplitudes attributed to the inspiratory phase and the other extreme amplitude of the circulation value out of the amplitudes attributed to the expiratory phase, and
    - for deriving from the amplitude variation between the extreme amplitudes of the two phases of the respiratory cycle whether the given hemodynamic load by said ventilation support is too high.

2. Device according to claim 1, **characterized by** the calculation unit being adapted for determining one maximum and one minimum amplitude each during the inspiratory and during the expiratory phase, and for determining both amplitude variations between the maximum amplitude during one phase and the minimum amplitude during the other phase.

3. Device according to claim 2, **characterized by** the calculation unit being adapted for selecting out of the determined amplitude variations the larger absolute value with its signs considered as relevant value for the hemodynamic load of the person.

4. Device according to one of claims 1 to 3, **characterized by** display means for displaying the amplitude variation and/or the derivation from the amplitude variation indicating whether the hemodynamic load by ventilation of the person is too high or not and/or a tendency of at least one of these parameters.

5. Device according to one of claims 1 to 4, **characterized by** the calculation device being adapted for deciding on the basis of the variation of said amplitudes of the circulation values for a respiratory cycle whether the end-expiratory pressure PEEP of the ventilator can be increased or must be reduced.

6. Device according to one of claims 2 to 5, **characterized by** the larger variation, in terms of an absolute value with signs considered of the amplitudes of the circulation value being taken into consideration when deciding on the PEEP.

7. Device according to one of claims 5 or 6, **characterized by** the decision regarding the PEEP being based on a median ZAVmed of several, in particular eleven ZAV variations of the amplitudes ZA of the circulation value Z of consecutive respiratory cycles considered for the decision regarding the PEEP.

8. Device according to one of claims 1 to 7, **characterized by** the variation ZAV of the amplitude ZA of the circulation value Z of a respiratory cycle is normalized, in particular by the following formulas:

$$ZAV1 = 2\frac{ZA\,insp - max - \; ZA\,exp - min}{ZA\,insp - max \; + \; ZA\,exp - min}$$

or

$$ZAV2 = 2\,\frac{ZA\,insp-min\,-\,ZA\,exp-max}{ZA\,insp-min\,+\,ZA\,exp-max}$$

wherein ZA insp-max is the maximum amplitude of a circulation value during the inspiratory phase, ZA exp-min the minimum amplitude of a circulation value during the expiratory phase, ZA insp-min the minimum amplitude of a circulation value during the inspiratory phase and ZA exp-max the maximum amplitude of a circulation value during the expiratory phase.

9. Device according to one of claims 1 to 8, **characterized by** the circulation value Z being a pulse-oximetry-plethysmographic measuring value POP.

10. Device according to one of claims 1 to 9, **characterized by** the detection means for detecting the respiratory phases being integrated in a ventilator (11).

11. Device according to claim 10, **characterized by** the device comprising the ventilator and a pulse oximeter (25) connected to the ventilator.

12. Device according to claim 11, **characterized by** the calculation device being adapted for determining only a maximum and a minimum value of the amplitudes during several inspiratory and expiratory phases and for using the variation of the amplitudes during these breaths for the evaluation.

13. Device according to one of claims 1 to 12, **characterized by** the calculation device being adapted

- for attributing the amplitudes of several respiratory cycles to the inspiratory phases or the expiratory phases, and
- for determining the one extreme amplitude of the circulation value out of the amplitudes attributed to the several inspiratory phases and the other extreme amplitude of the circulation value out of the amplitudes attributed to the several expiratory phases.

14. Device according to one of claims 1 to 13, **characterized by** the device comprising a monitoring device, a pulse oximeter connected to the latter and detection means connected to the monitoring device for detecting the respiratory phases.

15. Method, comprising:

- detecting the inspiratory phase and the expiratory phase of each respiratory cycle of a person by means of detection means (23, 25),
- detecting at least one minimum and at least one maximum amplitude of a circulation value during one single respiratory cycle,
- calculating a variation of the amplitudes of the circulation value occurring during one respiratory cycle,
**characterized by**
- the attribution of each amplitude of the circulation value to the inspiratory phase or the expiratory phase by means of a calculator device, and
- the determination by means of the calculation device of the one extreme amplitude of the circulation value out of the amplitudes attributed to the inspiratory phase and the other extreme amplitude of the circulation value out of the amplitudes attributed to the expiratory phase.

**Revendications**

1. Dispositif (11) avec des moyens de détection (23, 25) appropriés pour détecter

- la phase d'inspiration et la phase d'expiration de chaque cycle respiratoire d'une personne (13) sous assistance respiratoire,
- au moins une amplitude minimale et une amplitude maximale d'une valeur de circulation pendant un seul cycle respiratoire,

et avec un dispositif de calcul pour calculer

- une variation des amplitudes de la valeur de circulation rencontrée pendant ledit cycle respiratoire,

**caractérisé par**

le dispositif de calcul étant adapté

- pour attribuer chaque amplitude de la valeur de circulation ou à la phase d'inspiration (I) ou à la phase d'expiration (E), et

- pour déterminer ladite une amplitude extrême de la valeur de circulation parmi les amplitudes attribuées à la phase d'inspiration et l'autre amplitude extrême parmi les amplitudes attribuées à la phase d'expiration, et

- pour déduire de la variation d'amplitude entre les amplitudes extrêmes pendant les deux phases si la charge hémodynamique donnée par l'assistance respiratoire est trop grande.

2. Dispositif selon la revendication 1, **caractérisé par** l'unité de calcul étant adaptée pour déterminer, et pendant la phase d'inspiration et pendant la phase d'expiration, respectivement une amplitude minimale et une amplitude maximale, et pour déterminer les deux variations d'amplitude entre l'amplitude maximale pendant une phase et l'amplitude minimale pendant l'autre phase.

3. Dispositif selon la revendication 2, **caractérisé par** l'unité de calcul étant adaptée pour déterminer parmi les variations d'amplitude déterminées celle avec la plus grande valeur absolue prenant en compte les signes comme valeur applicable pour la charge hémodynamique d'une personne.

4. Dispositif selon une des revendications 1 à 3, **caractérisé par** des moyens d'affichage pour l'affichage de la variation d'amplitude et/ou la dérivation de la variation d'amplitude, indiquant si la charge hémodynamique par l'assistance respiratoire pour la personne est trop grande ou pas et/ou indiquant une tendance d'au moins une de ces valeurs.

5. Dispositif selon une des revendications 1 à 4, **caractérisé par** l'unité de calcul étant adaptée pour déterminer sur la base de la variation des amplitudes des valeurs de circulation d'un cycle respiratoire si une pression de fin de l'expiration (PEP) de l'appareil respiratoire peut être augmentée ou doit être réduite.

6. Dispositif selon les revendications 2 et 5, **caractérisé par** la variation des amplitudes de la valeur de circulation ayant une plus grande valeur absolue prenant en compte les signes étant prise en considération lors de la décision concernant la PEP.

7. Dispositif selon une des revendications 5 ou 6, **caractérisé par** la décision concernant la PEP étant basée sur une médiane ZAVmed de plusieurs, surtout onze, variations ZAV des amplitudes ZA de la valeur de circulation Z de cycles respiratoires consécutifs pris en considération lors de la décision concernant la PEP.

8. Dispositif selon une des revendications 1 à 7, **caractérisé par** la variation ZAV de l'amplitude ZA de la valeur de circulation Z d'un cycle respiratoire étant normalisée, en particulier selon les formules:

$$ZAV1 = 2\,\frac{ZA\,insp - max\ -\ ZA\,exp - min}{ZA\,insp - max\ +\ ZA\,exp - min}$$

ou

$$ZAV2 = 2\,\frac{ZA\,insp - min\ -\ ZA\,exp - max}{ZA\,insp - min\ +\ ZA\,exp - max}$$

ZA insp-max étant l'amplitude maximale d'une valeur de circulation pendant la phase d'inspiration, ZA exp-min étant l'amplitude minimale d'une valeur de circulation pendant la phase d'expiration, ZA insp-min étant l'amplitude minimale d'une valeur de circulation pendant la phase d'inspiration et ZA exp-max étant l'amplitude maximale d'une valeur de circulation pendant la phase d'expiration.

9. Dispositif selon une des revendications 1 à 8, **caractérisé par** la valeur de circulation Z étant une valeur de mesure d'oxymétrie à impulsion pléthysmographique POP.

10. Dispositif selon une des revendications 1 à 9, **caractérisé par** les moyens de détection pour détecter les phases respiratoires étant intégrés dans un appareil respiratoire (11).

11. Dispositif selon la revendication 10, **caractérisé par** le dispositif comprenant l'appareil respiratoire et un oxymètre d'impulsion (25) connecté à l'appareil respiratoire.

12. Dispositif selon la revendication 11, **caractérisé par** le dispositif de calcul étant adapté pour trouver, pendant plusieurs phases d'inspiration et phases d'expiration, seulement une valeur maximale et une valeur minimale des amplitudes et pour utiliser la variation des amplitudes pendant ces souffles pour l'évaluation.

13. Dispositif selon une des revendications 1 à 12, **caractérisé par** le dispositif de calcul étant adapté

- pour attribuer les amplitudes de plusieurs cycles respiratoires aux phases d'inspiration et d'expiration et
- pour déterminer ladite une amplitude extrême de la valeur de circulation parmi les amplitudes associées audites plusieurs phases d'inspiration et pour déterminer l'autre amplitude extrême de la valeur de circulation parmi les amplitudes associées audites plusieurs phases d'expiration.

14. Dispositif selon une des revendications 1 à 13, **caractérisé par** le dispositif comprenant un dispositif de surveillance, un oxymètre d'impulsion connecté à ce dernier et des moyens de détection connectés au dispositif de surveillance pour détecter les phases respiratoires.

15. Méthode comprenant les mesures suivantes:

- détecter la phase d'inspiration et la phase d'expiration de chaque cycle respiratoire d'une personne par des moyens de détection (23, 25),
- détecter au moins une amplitude minimale et une amplitude maximale d'une valeur de circulation pendant un seul cycle respiratoire,
- calculer une variation des amplitudes de la valeur de circulation ayant lieu pendant un cycle respiratoire,
**caractérisée par**
- l'attribution de chaque amplitude de la valeur de circulation à la phase d'inspiration ou à la phase d'expiration au moyen d'un dispositif de calculateur, et
- la détermination de ladite une amplitude extrême de la valeur de circulation parmi les amplitudes attribuées à la phase d'inspiration et ladite autre amplitude extrême de la valeur de circulation parmi les amplitudes attribuées à la phase d'expiration au moyen du dispositif de calcul.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1813187 A

- WO 03077854 A

**In der Beschreibung aufgeführte Nicht-Patentliteratur**

- Changes in Arterial Pressure during Mechanical Ventilation. *Frederic Michard, Anesthesiology,* 2005, vol. 103, 419-428
- **MAXIME CANNESSON et al.** Does the Pleth Variability Index Indicate the Respiratory-Induced Variation in the Plethysmogram and Arterial Pressure Waveforms?. *ANESTHESIA & ANALGESIA,* April 2008, vol. 106 (4), 1190-1194

- Using heart-lung interactions to assess fluid responsiveness during mechanical ventilation. *Crit Care,* 01. September 2000, vol. 4, 282-289